(19)

**Europäisches Patentamt**

**European Patent Office**

**Office européen des brevets**

(11) **EP 4 134 964 A1**

(12) **EUROPEAN PATENT APPLICATION**

(43) Date of publication:
**15.02.2023 Bulletin 2023/07**

(21) Application number: **21190794.4**

(22) Date of filing: **11.08.2021**

(51) International Patent Classification (IPC):
**G16H 10/40** *(2018.01)*

(52) Cooperative Patent Classification (CPC):
**G16H 10/60; G16H 10/40**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Beckman Coulter Inc.**
**Brea, California 92821 (US)**

(72) Inventor: **STENNIS, Marcus**
**82131 Gauting (DE)**

(74) Representative: **Müller-Boré & Partner**
**Patentanwälte PartG mbB**
**Friedenheimer Brücke 21**
**80639 München (DE)**

<u>Remarks:</u>
Amended claims in accordance with Rule 137(2) EPC.

(54) **COMPUTER-IMPLEMENTED METHOD IN THE FIELD OF CLINICAL TESTS OF BIOLOGICAL SAMPLES**

(57) Summarizing the invention, a computer-implemented method is provided. The method comprises: accessing, by a first computing device, test data and instrument data, wherein: the test data comprise information specifying at least one clinical test to be carried out on a biological sample of a subject, the instrument data comprise information specifying a state and/or a configuration of a set of laboratory instruments, and at least one laboratory instrument of the set of laboratory instruments is configured to carry out the at least one clinical test; generating, by the first computing device, sample-collecting data by using at least the test data and the instrument data, wherein: the sample-collecting data comprise at least one of a sample volume requirement and a sample container requirement, the sample volume requirement comprises one or more suitability constraints on a volume of the biological sample, and the sample container requirement comprises one or more constraints on a container in which the biological sample is to be held; providing, by the first computing device, the sample-collecting data to a phlebotomy agent.

Figure 2

EP 4 134 964 A1

**Description**

Technical Field

The present invention relates to the field of biological samples handling and/or testing.

Background

Analytical and clinical laboratories typically comprise several laboratory instruments that are configured to automatically carry out one or more clinical tests on biological samples (such as blood samples). In many cases, these instruments are integrated in automated laboratory systems that comprise also means for automatically transporting the to-be-tested biological samples from one instrument to another.

Accordingly, a laboratory instrument receives a biological sample usually held in a container and processes the biological sample to perform a given clinical test. The processing of the biological sample may be hindered by sub-optimal or inadequate features of the container and its sample, in that one or more features of the sample and/or of the container may lead to incorrect results of the clinical test or to a halt of the processing. Consequently, the processing time and the reliability of the clinical test may be negatively affected.

Summary

It is an object of the invention to provide means for improving the reliability and efficiency of the processing of biological samples for clinical test.

The achievement of this object in accordance with the invention is set out in the independent claims. Further developments of the invention are the subject matter of the dependent claims.

According to a first aspect, a computer-implemented method is provided. The method comprises:

- accessing, by a first computing device, test data and instrument data, wherein:

    o the test data comprise information specifying at least one clinical test to be carried out on a biological sample of a subject,
    o the instrument data comprise information specifying a state and/or a configuration of a set of laboratory instruments, and
    o at least one laboratory instrument of the set of laboratory instruments is configured to carry out the at least one clinical test;

- generating, by the first computing device, sample-collecting data by using at least the test data and the instrument data, wherein:

    o the sample-collecting data comprise at least one of a sample volume requirement and a sample container requirement,
    o the sample volume requirement comprises one or more suitability constraints on a volume of the biological sample, and
    o the sample container requirement comprises one or more constraints on a container in which the biological sample is to be held;

    - providing, by the first computing device, the sample-collecting data to a phlebotomy agent.

According to a second aspect, a computer-implemented method is provided. The method comprises:

- accessing, by a second computing device, sample-collecting data generated based on at least test data and instrument data, wherein:

    o the test data comprise information specifying at least one clinical test to be carried out on a biological sample of a subject,
    o the instrument data comprise information about a state and/or a configuration of a set of laboratory instruments, and at least one laboratory instrument of the set of laboratory instruments is configured to carry out the at least one clinical test;
    o the sample-collecting data comprise at least one of a sample volume requirement and a sample container requirement,

o the sample volume requirement comprises one or more suitability constraints on a volume of the biological sample, and

o the sample container requirement comprises one or more constraints on a container in which the biological sample is to be held;

- providing, by the second computing device, the sample-collecting data to a phlebotomy agent.

Unless specified otherwise, the following discussion applies to both aspects described above. A computing device, e.g. the first computing device or the second computing device, may comprise at least one memory and at least one processor. A computing device may also comprise one or more input/output units.

The first computing device accesses test data and instrument data. In the present disclosure, "accessing, by a computing device (e.g. the first computing device), data" may comprise retrieving the data e.g. from the at least one memory of said computing device or from a remote data storage (a database, a secondary memory, or the like). In particular, said computing device retrieves data in response to a user command and/or to a notification sent by another computing device.

Additionally or alternatively, "accessing, by a computing device, data" may comprise receiving the data, e.g. from a user or another computing device. The two options are not mutually exclusive. For instance, accessing, by a computing device, data may comprise receiving the data, storing the data in the memory of said computer device and retrieving the data by accessing said memory.

The step of accessing the instrument data may be performed in response to a request received by another computing device, wherein the request may contain the test data. In other words, the first computing device may receive the test data (i.e. passively access the test data) and, in response, may access the instrument data, which may be stored in the memory of the first computing device. The subsequent steps of generating and providing sample-collecting data (discussed below) may also be part of the response to the request.

The test data contain information identifying at least one clinical test to be carried out on the biological sample of the subject. In particular, the subject may be a human or an animal subject. The biological sample of the subject may be a sample of a bodily fluid of the subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing the at least one clinical test. For instance, blood serum, blood cells and blood plasma are components of a blood sample.

A clinical test may comprise one or more procedures that, when carried out on the biological sample or on a component thereof, allow for estimating the value of a parameter, e.g. a clinical parameter. In particular, a clinical test may comprise physical, biological, optical, mechanical, immunological, and/or chemical procedures.

The information in the test data specifies one or more clinical tests, or, in other words, a set of clinical tests (wherein a set can also comprise only one element). Indeed, in some cases, a plurality of tests can be performed on a given sample, provided that the volume is sufficient, as discussed in more detail below.

The test data may comprise, for each clinical test, an identifier of said test, wherein said identifier identifies said test and, hence, contains information specifying said test. The identifier may be an alphanumeric string. For instance, the alphanumeric string "TSH" uniquely identifies the thyroid-stimulating hormone test and the string "Lp-PLA2" uniquely identifies the Lipoprotein-Associated Phospholipase A2 test. Exemplarily, the test data may comprise only or at least the identifier(s) of the test(s).

The instrument data comprise information indicative of the configuration and/or of the state of the set of laboratory instruments. A laboratory instrument may be an analyser, i.e. an apparatus configured to carry out one or more clinical tests on a biological sample. In this case, the set of laboratory instruments may a set of analysers.

The configuration of the set of laboratory instrument may refer to the capabilities of at least an instrument, e.g. of each instrument of the set, and, in particular, to features concerning the functioning thereof (i.e. the performing of tests). In particular, the information specifying the configuration of a set of laboratory instruments may comprise information indicative of the configuration of at least one laboratory instrument, e.g. of each laboratory instrument of the set of laboratory instruments.

In particular, laboratory instruments are configurable in different ways to perform different clinical tests. Information indicative of the configuration of laboratory instrument may comprise information indicative of the clinical tests that said laboratory instrument is configured to carry out. The latter information may be specified by one or more test identifiers associated with said laboratory instrument. As discussed above, a test identifier may be an alphanumeric string that uniquely determines the clinical test. For instance, if the laboratory instrument uses a reagent to carry out a clinical test and this reagent is exclusively used to carry out said test, the alphanumeric string identifying said test (in the instrument data) may consist of or comprise the name of said reagent.

Alternatively, or in conjunction with the above, information indicative of the configuration of a laboratory instrument may comprise information indicative, for each test that can be carried out by said instrument, of the component of the biological sample that is needed by said instrument to carry out said each test. For instance, if the biological sample is a blood sample, depending on the instrument and the test, the component may be blood cell, blood plasma, or blood serum.

Alternatively, or in conjunction with the above, information indicative of the configuration of a laboratory instrument may comprise, for each test of the tests that said instrument is configured to carry out, information indicative of the minimum amount of sample volume needed for performing each test by using said instrument.

Alternatively, or in conjunction with the above, information indicative of the configuration of a set of laboratory instruments may comprise information indicative of the dead volume of said laboratory instrument. In particular the dead volume of an instrument is the minimal amount of sample that said instrument requires in order to properly aspirate a suitable amount of the sample from the container in which the biological sample is held. In particular, the dead volume is the minimal volume that allows the pipette to aspirate the suitable amount of the sample without aspirating air. The dead volume may depend on the range of motion of a pipette of the instrument in the z-direction and/or on the type of container that said instrument may handle. Alternatively, or in conjunction with the above, information indicative of the configuration of a laboratory instrument may comprise information indicative of the types of container that are compatible with, e.g. can be handled by, the instrument. In particular, said information may be specified by a container-type identifier which is associated to and uniquely identifies a container type. In particular, a container-type identifier may be an alphanumeric string that uniquely identifies the type of container associated thereto.

A type of container is in particular a category of containers that is characterised by at least a shared property, wherein said property can be used to assess whether a specific container belongs to the category or not. For example, shared properties may be the container shape, the container dimensions, the container volume, or the like. For instance, if the container is a tube, a type of container may be characterised by the longitudinal length of the tube, by the shape of the tube cross section, by the transversal dimensions of the tube (e.g. the diameter of the cross section thereof), by the volume of the tube and/or by the type of cap that closes the container (e.g. shape or colour of the cap).

The state of the set of laboratory instrument may refer to a condition of at least an instrument, e.g. of each instrument of the set, and/or, in particular, to stationary features of the instrument (e.g. the identity and/or the physical properties thereof). In particular, the information indicative of the state of a set of laboratory instruments may comprise information indicative of the state of at least a laboratory instrument, e.g. of each laboratory instrument, of the set of laboratory instruments.

Information indicative of the state of a laboratory instrument may comprise information indicative of the identity of said laboratory instrument. The latter information may be specified by an instrument identifier which is associated to and uniquely identifies said laboratory instrument. In particular, an instrument identifier may be an alphanumeric string that uniquely identifies the laboratory instrument associated thereto, e.g. the serial number of the laboratory instrument.

Alternatively or additionally, information indicative of the state of a laboratory instrument may comprise information indicative of the availability of said laboratory instrument, e.g. whether said instrument is functioning or not.

Exemplarily, the instrument data may comprise only or at least the following pieces of information for each laboratory instrument in the set: instrument identifier, dead volume, set of tests that can be performed by said instrument, minimum sample volume for each test that can be performed by said instrument, type of component that said instrument needs in order to carry out each test that can be performed by said instrument, and type(s) of container compatible with said laboratory instrument.

The instrument data may provide current information about the laboratory instruments, e.g. information which is regularly updated. For example, the time interval between two consecutive updated may be adjustable e.g. by an operator. In particular, the step of accessing instrument data may comprise the step of obtaining and/or receiving at least a portion of the instrument data from at least a laboratory instrument of the set of laboratory instruments.

The method further comprises generating sample-collecting data by using at least the test data and the instrument data. The sample-collecting data may comprise information relevant to the collection of the sample, i.e. taking the sample from the subject and storing the sample in a sample container. In particular, the sample-collecting data may comprise information necessary for collecting the sample.

The sample-collecting data comprise at least one of a sample volume requirement and a sample container requirement, i.e. either one or both. A requirement is *per se* a piece of information but it is associated to a specific aspect or entity, such as the sample volume or the sample container. Accordingly, a requirement is a piece of information that expresses a necessary characteristic for the given aspect or entity, wherein the necessity arises from the context. In the present context of clinical tests, the sample volume requirement and the sample container requirement prescribe characteristics of the sample volume and of the sample container that are necessary for performing the at least one clinical test on the sample. These requirements can be implemented e.g. by a phlebotomist agent.

In particular, the sample volume requirement may be or comprise information indicative, e.g. to the phlebotomist agent, of the amount of volume suitable for carrying out each clinical test of the set of clinical tests on the biological sample e.g. by using the set of laboratory instruments. For instance, said requirement may specify at least the minimum amount of sample volume required for carrying out each clinical test of the set of clinical tests on the biological sample e.g. by using the set of laboratory instruments.

The sample volume requirement comprises one or more suitability constraints specifying at least a volume, $\overline{V}$, of the biological sample. In particular, said volume indicates an amount of biological material, e.g. the minimum amount thereof, that shall be collected from the subject e.g. in order to carry out each clinical test of the set of clinical tests on the biological sample by using the set of laboratory instruments.

As mentioned above, the sample-collecting data comprise, in addition or alternatively to the sample volume requirement, a sample container requirement. The sample container requirement comprises one or more constraints on a container in which the biological sample is to be held. In particular, it may constrain one or more physical features of the container and/or one or more functional features of the container into which the biological sample is to be collected for carrying out each clinical test of the set of clinical tests e.g. by using the set of laboratory instruments. Functional features of the container are, for example, features related to the use of the container in the processing of the sample. For instance, a functional feature is the presence of a reagent and the quantity thereof.

The sample container requirement may be or comprise information indicative, e.g. to the phlebotomy agent, of a type of container into which the biological sample is to be collected for carrying out each clinical test of the set of clinical tests e.g. by using the set of laboratory instruments. For example, this type of container is a type of container compatible with the laboratory instruments that are expected to carry out each clinical test of the set of clinical tests.

Exemplarily, the first computing device may store the sample-collecting data in its memory.

After generating the sample-collecting data, the first computing device provides them to a phlebotomy agent. The phlebotomy agent may be a human being which, in particular, is trained to collect, e.g. draw, biological samples (e.g. a phlebotomist, a doctor, a nurse, or the like). The phlebotomy agent may be a phlebotomy device (e.g. a robot) configured to collect, e.g. draw, biological samples. In particular, the information comprised in the sample-collecting data assists the phlebotomy agent in collecting, e.g. drawing, the biological sample. In particular, the phlebotomy device is an "intelligent" device comprising a computing device, e.g. at least a processor and at least a memory. The phlebotomy device may comprise drawing means (e.g. a robotic arm) configured to draw the biological sample from the individual. The drawing means may be operatively connected with the computing device of the phlebotomy device, said computing device being configured to control the drawing means.

The phlebotomy agent may process, e.g. by using the second computing device, the sample-collecting data to ascertain a requirement on the volume of the biological sample and/or to ascertain the type of container into which the biological sample is to be collected for carrying out each clinical test of the set of clinical tests e.g. by using the set of laboratory instruments.

In the present disclosure, "providing, by the first computing device, the sample-collecting data to the phlebotomy agent" may refer to making said data available to the agent. In one example, the step of providing the sample-collecting data to the phlebotomy agent may comprise providing said data to a computing device that may be accessed by the phlebotomy agent. For example, said computing device may be the second computing device. In particular, if the phlebotomy agent is a phlebotomy device, providing the sample-collecting data to the phlebotomy agent may comprise providing said data to the computing device of the phlebotomy agent. In another example, providing data to the phlebotomy agent may comprise displaying said data on a screen of the first computing device. For instance, these data are displayed on the screen of the first computing device if the phlebotomy agent is a human being which has access to, e.g. can look at, the screen of the first computing device.

The sample-collecting data are generated by using the instrument data and, thus, relate to the state and/or configuration of the set of laboratory instruments that are to carry out the clinical test(s). The information comprised in the sample-collecting data enables the phlebotomy agent to provide to the laboratory instruments a suitable amount of biological sample and/or suitable sample-holding containers, thereby preventing problems related to the handling of wrong amounts of samples and/or unsuitable types of containers.

Specifically, by ensuring that the amount of collected sample is sufficient for the test(s), it can be prevented that the processing of the sample is delayed and/or faulty. Indeed, if the amount of volume is insufficient, the automated laboratory system may, for instance, take actions such as notifying an operator, prioritise some tests over other tests, and/or move the sample in a "error" area of the system. These actions are detrimental to the processing time of the system, may lead to incomplete or unsatisfactory test results and/or to human errors in the handling of samples.

Moreover, in these cases, the ordered tests may be completed only by obtaining another biological sample from the subject, which may unduly stress the subject body, delay the provision of the test results, and/or increase the workload of the phlebotomist agent and/or the laboratory instruments/system. Accordingly, providing a sample volume requirement prevents unduly stress to the subject body by avoiding the necessity of a second draw (in case of insufficient volume) or the occurrence of an overdraw (i.e. a volume unnecessarily high). An overdraw may be particularly undesirable for young children and sick/elderly patients.

Similarly, providing a sample container requirement prevents the occurrence of halts/delays in the processing and/or incorrect performing of the tests. For example, if a container cannot be correctly handled by a laboratory instrument, the instrument may provide no, unreliable, inconclusive or incorrect results, as e.g. the instruments may aspirate an incorrect sample volume and consequently process an unsuitable amount of biological sample. In another example, the sample may be aliquoted or transferred to a suitable container, which not only delays the processing, but may lead to a contamination of the sample and/or the instruments.

To summarize, thanks to the sample-collecting data, the samples are tailored to the actual execution of the clinical tests, so that delays or faults of the clinical tests can be avoided.

In a particular example, the test data may further comprise information specifying the priority of each test of the set of clinical tests, i.e. whether said each test is a STAT test or not. Alternatively, or in conjunction with the above, the test data may comprise, for each test of the set of clinical tests, information specifying the bodily fluid (e.g. blood) and/or the component thereof (e.g. blood serum or blood components) required. Alternatively or additionally, the test data may comprise indications about how to prepare the subject for collecting a sample for the specific test.

In a particular example, at least one laboratory instrument of the set of laboratory instruments is configured to carry out the at least one clinical test. In particular, for each test of the set of clinical tests, there is at least a respective instrument of the set of laboratory instruments, said respective instrument being configured to carry out said test. For instance, each laboratory instrument of the set of laboratory instruments is configured to carry out at least one clinical test of the set of clinical tests. Alternatively, one or more laboratory instruments in the set of laboratory instruments may not be configured to carry out any of the tests in the set of clinical tests.

Exemplarily, if a set of laboratory instruments comprises a plurality of instruments, each laboratory instrument may be configured to perform a different set of clinical tests. In other words, a specific clinical test may be performed only by one laboratory instrument. In another example, two or more laboratory instruments may be configured to perform the same clinical test.

In some examples, the method according to the first aspect may further comprise the steps of accessing, by the first computing device, device data, wherein: the device data comprise information indicative of a state and/or configuration of a set of pre-analytical and/or post-analytical devices, and wherein the step of generating the sample-collecting data is carried out by using also at least the device data. In particular, the device data comprise information indicative of at least a device, e.g. each device of said set of devices.

A pre-analytical device is in particular a device configured to carry out one or more pre-analytical steps on biological samples to prepare said samples for being tested by laboratory instruments. For example, centrifuges, aliquoters and de-cappers are pre-analytical devices. In particular, a device configured to detect the level of a volume of a biological sample held in a container is a pre-analytical device. A post-analytical device is in particular, a device configured to carry out one or more post-analytical steps such as storing and/or refrigerating biological samples. For example, re-cappers and sample storage units are postanalytical devices. In the following, the term "processing device(s)" may refer to a pre-analytical and/or a post analytical device(s).

Information indicative of the configuration of a processing device may comprise information indicative of the processing steps that said device may carry out. For instance, if the processing device is a centrifuge, the information indicative of the configuration of the processing device specifies that said device may centrifuge the sample. Alternatively, or in conjunction with the above, the information indicative of the configuration of a processing device may comprise information indicative of the minimum amount of sample volume needed for performing the processing step. For instance, if the processing device is a device configured to detect the level of biological samples in the containers, information indicative of the configuration of the processing device specifies the minimum amount of volume that is detectable by the device.

Information indicative of a state of a processing device may comprise information indicative of the identity of said device, i.e. a device identifier which is associated to and uniquely identifies said device. Information indicative of the state of a processing device may comprise information indicative of the availability of said device, e.g. whether said instrument is functioning or not, and/or of the expected workload of said device.

Accordingly, the sample-collecting data may be generated by further using information on the state and/or configuration of pre- and/or post-analytical devices. Hence, the sample volume requirement and/or on the container type requirement may guarantee that the biological sample may be properly pre- and/or post-processed, thereby increasing the accuracy of the test results and/or the reproducibility thereof.

In a particular example, the set of instruments may be comprised in an automated laboratory system (hereinafter also referred to as "ALS"). In particular, an ALS is an assembly comprising a plurality of laboratory instruments and a computing device, e.g. the first computing device, wherein the computing device is operatively connected to these laboratory instruments. In particular, the computing device is configured to control each instrument of the plurality of laboratory instruments, by e.g. instructing said each instrument to carry out tests on biological samples.

The ALS may comprise transporting means, e.g. tracks, belts and/or tube carriers, for transporting tubes comprising biological samples to the laboratory instruments of the ALS for testing. In this case, in particular, the computing device of the ALS is also configured to control the transport of the biological samples, e.g. by controlling the transporting means. Moreover, the ALS may further comprise pre-analytical devices laboratory instruments, and/or post-analytical devices.

If the set of instruments is comprised in an ALS, the instrument data may comprise information indicative of a configuration and/or a state of the ALS (hereinafter also referred to as "ALS information"). In particular, ALS information may comprise information specifying how the laboratory instruments of the ALS are connected with one another and, optionally, with output and/or input regions of the ALS.

ALS information may comprise information indicative of the state a pre-analytical device, a post-analytical device and/or the transporting means of the ALS. In other words, the ALS information may comprise the information of the device data discussed above and/or information about the transporting means. For instance, information indicative of the state of the transporting means comprises information indicative of the availability of the transporting means e.g. whether at least a portion of the transporting means is functioning or not.

ALS information is in particular information suitable for generating a route plan for processing the biological sample by using the ALS. Exemplarily, the step of generating sample-collecting data may comprise generating the route plan by using the instrument data, e.g. the ALS information, and the set of clinical tests in the test data. In particular, the route plan specifies one or more laboratory instruments that, according to the route plan of the sample, are expected to carry out the test(s) of the set of clinical tests. In this case, the step of generating the sample-collecting data is carried out by using information indicative of the state and/or the configuration of those laboratory instruments of the ALS that are specified in the route plan.

In this example, the sample-collecting data are generated by using information indicative of the state and/or configuration of the ALS as a whole. In particular, said data are generated by using not only the state and/or configuration of the laboratory instruments, but also information indicative of the state and/or configuration of the pre-analytic devices configured to prepare the sample for testing as well as information indicative of the state and/or configuration of the transporting means configured to move the samples from one laboratory instrument to another. The ALS information allows for providing an accurate estimate of the laboratory instruments that will be actually used to carry out the test(s) of the set of clinical tests (e.g. according to the route plan), thereby increasing the accuracy of the generated sample-collecting data.

In a particular example, the step of generating the sample-collecting data may comprise selecting, by the first computing device, from the set of laboratory instruments one or more instruments, said one or more instrument being configured to carry out the at least one clinical test. In particular, the selecting of the laboratory instruments may be based on one or more selection criteria.

In some cases, the instrument data (in particular the information indicative of the configuration of a laboratory instrument) may comprise time information, said information being indicative, for at least one of the tests that can be carried out by said instrument, of the execution time of said test. The execution time is in particular the amount of time required by said instrument to carry out said test. In particular, in this case, the one or more selection criteria may be based on the time information, so that, for at least one test, the selection of the one or more instruments comprises selecting the laboratory instrument of the set of laboratory instruments that (i) is configured to carry out said test and (ii) has the smallest execution time for said test. This way, the processing time of the biological sample is reduced.

Additionally or alternatively, the one or more selection criteria may be based on other information in the instrument data. In particular, said one or more selection criteria may be based on information indicative of the minimum amount of sample volume needed for performing the clinical tests that can be carried out by said one or more laboratory instruments of the set of laboratory instruments. For example, in this case, for at least one test, the selection of the one or more instruments comprises selecting the laboratory instrument of the set of laboratory instruments that (i) is configured to carry out said clinical test and (ii) has the greatest minimum amount of sample volume needed for performing said clinical tests. This way, the amount of biological sample specified by the sample volume requirement is suitable for carrying out said test irrespective of the laboratory instrument that will be used to carry said test.

The instrument data (in particular the information indicative of the state of a laboratory instrument) may exemplarily comprise information indicative of the calibration state of the laboratory instrument, e.g. information specifying when the next calibration of the laboratory instrument is scheduled. Alternatively or additionally, the information indicative of the state of a laboratory instrument may comprise:

- workload information, said information being indicative of the expected workload for the instrument, e.g. of the number of samples in the instrument's queue at an estimated time at which the biological sample is ready for being analysed by the instrument; and/or
- an estimated time at which the instrument is expected to start processing the biological sample; and/or
- maintenance information, said information being indicative of the maintenance schedule of the instrument, e.g. when the next maintenance of the instrument is scheduled.

The estimated time at which the biological sample is ready for being analysed by the instrument may be based on a delivery time of the biological sample and/or on the sum of the delivery time and a pre-processing time of the biological sample. In particular, the delivery time of the biological sample may be the actual amount of time or the estimated amount of time needed to deliver the biological sample to the location of the instrument, e.g. of the set of instruments. For instance, the delivery time may be substantially equal to the transport time, i.e. to the amount of time needed to transport the biological sample from the location of the phlebotomy agent to the location of the instrument. In some cases, the delivery time may be the sum of the transport time and the amount of time needed to prepare the biological sample for delivery. The pre-processing time is in particular the amount of time needed to pre-process (e.g. centrifuge, aliquot, de-cap) the biological sample so that it can be appropriately analysed by the instrument, e.g. by each of instrument of the set of biological instruments.

The number of samples in the instrument's queue may be a number referring to actual sample tubes awaiting analysis e.g. according to an established current or future schedule. Alternatively or additionally, the number of samples in the instrument's queue may be estimated based on historical data of the instrument collected in the past and stored in a memory. For instance, the historical data may comprise information about an average number of samples received by the instrument at a given time of the day and/or on a given day of the week.

A given instrument may require a certain amount of time (hereinafter referred to as "test time") to carry out a test on a biological sample. Further, there may be a plurality of other samples in sample tubes to be analysed by said instrument before the biological sample in question, e.g. in a queue. Once a given biological sample is ready to be processed by the instrument (i.e. it has arrived at the laboratory and possibly undergone pre-processing), the estimated starting time for the processing of a given biological sample by the instrument depends on the test time and the number of samples in the queue before said sample.

In particular, information indicative of the maintenance schedule of the instrument consists of information suitable for estimating when the maintenance of the instrument shall be carried out. For example, if the laboratory instrument carries out a test by using a reagent, the maintenance information may comprise or be based on information specifying the amount of reagent currently contained in the instrument, the workload information, and the amount of reagent used to carry out each test.

Accordingly, the generation of the sample-collecting may be based on the knowledge of the maintenance schedule and/or the workload of the instruments and, hence, may be based on the state and/or configurations of the instruments with a relatively low workload and/or for which no maintenance is needed. This way, the amount of volume of the sample and/or the container into which said sample is collected are suitable for carrying out the clinical test(s) by using these instruments, thereby decreasing the processing time of the sample and/or increasing the reliability of the test results.

In this case, the selection criteria may be based on the workload information and/or on the maintenance information, so that, for at least one test, the selection of the one or more instruments comprises selecting the laboratory instrument of the set of laboratory instruments that (i) is configured to carry out said test and (ii) has the smallest workload and/or does not have any maintenance scheduled in the future interval of time.

In a particular example, the one or more suitability constraints of the sample volume requirement may comprise a volume value and/or a volume range. A suitability constraint comprising the numerical value $V_{re}$ as volume value may prescribe a specific quantity for the biological material. Accordingly, the piece of information $V_{re}$ may be construed as a requirement that the volume of taken biological material must be equal to $V_{re}$ within tolerance margins, e.g. $V_{re} \pm 1\%$ or $Vre \pm 5\%$. Similarly, in the case of a volume range, the suitability constraints may comprise or consists of one or two numerical values, e.g. $V_{low}$ and/or $V_{upp}$, and indicate that the amount of the volume of the sample shall lie between $V_{low}$ and $V_{upp}$, e.g. shall be substantially equal to or greater than $V_{low}$ and/or substantially equal to or lower than $V_{upp}$. Exemplarily, the suitability constraint may provide a lower limit, $V_{low}$, for the sample volume.

Exemplarily, the step of generating sample-collecting data may comprise determining the volume of biological sample $\overline{V}$ by using at least the test data and the instrument data. For instance, the first computing device may check the test data, in particular, the identifier(s) of the set of clinical tests, against the instrument data, in particular the sets of tests that can be performed by each laboratory instrument. Accordingly, the first computing device may identify which instrument(s) can carry out the requested clinical test(s) and which component of the biological samples are needed to carry out said tests.

In case of more than one laboratory instrument configured to perform a given clinical test, the first computing device may use other pieces of information of the instrument data to select a particular instrument, e.g. whether it is functioning, the expected occupancy, and so on. In some cases, as mentioned above, if a plurality of laboratory instruments are configured to perform a given clinical test, the first computing device may select the instrument that (i) is configured to carry out said given test and (ii) has the greatest minimum amount of sample volume needed for performing said given clinical test.

The set of clinical tests may comprise N tests to be performed on the biological sample. In particular, in order to perform all the tests of the set of tests, C components of the biological sample are needed and, more particularly, $n_s$ tests have to be performed on the component $s$ by using $m_s$ instruments of the set of laboratory instruments. The positive

integers $N, C, n_s$ and $m_s$ may be equal to or greater than 1 and $\sum_{s=1}^{C} n_s = N$. Each instrument $k$ of the $m_s$ instruments performs $p_{k,s}$ tests of the set of clinical tests on the component $s$, wherein $\sum_{k=1}^{m_s} p_{k,s} = n_s$.

The first computing device may access the instrument data and retrieve the dead volume $DV_k$ of the instrument $k$. Moreover, for each clinical test, $j$, carried out by the instrument $k$ of the $m_s$ instruments by using the component $s$ of the biological sample, the first computing device may look up in the instrument data the minimum amount of volume of the component $s$, $V_{j,k,s}$.

The first computing device estimates the minimum amount volume, $V_s$, of the component $s$ that is suitable for carrying out the $n_s$ tests on the component $s$. In particular, $V_s$ may be estimated by using the following equation:

$$V_s = \frac{1}{w_s} \sum_{k=1}^{m_s} \left( DV_k + \sum_{j=1}^{p_{k,s}} V_{j,k,s} \right). \qquad (1)$$

The positive numerical value $w_s$ is associated to the component $s$ and is lower than or equal to 1. Said numerical value takes into account the fact that, in some cases, the component $s$ may be only a fraction of the biological material of the sample as collected. For instance, typically, blood plasma amounts to about 55% of the human blood volume. Hence, if the biological sample is a human blood sample and the component $s$ is blood plasma, $w_s$ may be lower than or equal to 0.55, e.g. equal to 0.45 or 0.50.

For each component s, the numerical value $w_s$ may be comprised in composition data that e.g. are stored in the memory of the first computing device. In particular, the first computing device may access the memory to retrieve the composition data and estimate $V_{,s}$ by using equation (1). Accordingly, the step of generating the sample-collecting data may also be based on the composition data. In some cases, the computation may factor in a safety leeway, e.g. 5% or 10% of the value obtained from the equation (1) or a constant addendum.

If C components of the biological sample are needed to perform the tests of the set of clinical tests, the volume of biological sample $\overline{V}$ may be estimated from the following equation:

$$\overline{V} = \sum_{s=1}^{C} V_s. \qquad (2)$$

In some examples, the one or more suitability constraints on the volume of the biological sample comprise information specifying the value of the $\overline{V}$ volume given by equation (2).

Typically, if C components of the biological sample are needed to perform the tests of the set of clinical tests, before processing, the biological sample shall be distributed into at least C containers. In some cases, the biological sample may be placed in one container, e.g. by the phlebotomist agent, and aliquoting may take place later on, e.g. at the laboratory. Alternatively, the biological sample may be split from the onset (i.e. right after being collected) into a plurality of containers, e.g. into C containers.

In some examples, the sample volume requirement may comprise information indicative of the number of containers into which the biological sample is to be collected, and information indicative of the amount of volume of the biological sample to be collected in each of these containers. In other words, the sample-collecting data may specify the number of portions (hereinafter also referred to as: "sub-volumes"), into which the biological sample shall be split, and the amount of volume of each of said portions. In this context, the volume of the biological sample may be considered as comprising the plurality of sub-volumes, wherein each sub-volume is configured to be held in a respective container. Accordingly, the suitability constraints on the volume of the biological sample may comprise suitability constraints on each sub-volume.

For example, if the biological sample is to be split into C containers, the sample volume requirement may specify the values $V_1, ..., V_C$ given by equation (1) for the sub-volumes. Alternatively, the sample volume requirement may specify the value of $\overline{V}$ defined by equation (2) and the value of $(C-1)$ of the C sub-volumes $V_1, ..., V_C$, given by equation (1), e.g. the value of the sub-volumes $V_1, ..., V_{C-1}$. In this case, in particular, the phlebotomist agent and/or the second computing device may determine the sub-volume to be held in the $C$ - $th$ container by solving equation (2) for $V_C$.

For instance, two sub-volumes may be required. In an example in which the total volume may be drawn all at once from the subject, the constraint on the sample volume $\overline{V}$ may be useful to a phlebotomy agent for a reading on a syringe used for taking the biological material, while one of the constraints on a sub-volume may be used when partitioning the volume in one container, with the "leftover" being put in the other container. In another example in which the biological material has to be taken twice from a subject, the two constraints on the sub-volumes may be sufficient for the phlebotomy agent.

The necessity of splitting the biological sample into several containers may also arise if a plurality of tests is performed by different laboratory instruments, which can handle incompatible types of containers. Another possibility may be that the quantity of biological material to be collected is so large (e.g. for several clinical tests) that it cannot be contained in any single container.

Beyond this incompatibility and volume issues, the necessity of splitting the biological sample in several containers may also arise if, for example, the set of laboratory instruments does not comprise any instrument that can carry out a specific test in the set of clinical tests, so that part of the sample may need to be sent to a different laboratory. In this case, the sample-collection data may comprise information specifying the location into which each of the container shall be sent. This way, the need to carry out the tests at different locations can be already dealt with at the phlebotomy agent location, thereby parallelising, at least in part, the processing of the sample and, hence, reducing its processing time.

In a particular example, the sample volume requirement may be further based on the sample container requirement (if present), besides being based on the test data and the instrument data. For instance, a volume upper limit may be dictated by the maximum volume that the container that fulfils the sample container requirement (discussed below) can hold. In another instance, the diameter of the container and, thus, the distribution of the sample volume in the container and not just its absolute value, may affect the amount of volume that will be aspirated by a pipette of the instrument. Accordingly, the sample volume may have to be greater or smaller depending on the diameter of the container.

Exemplarily, the one or more constraints of the sample container requirement comprise a type of container. In particular, the one or more constraints of the sample container requirement comprise information indicative of at least a type of container that is compatible with, e.g. can be handled, by the instruments that are expected to carry out each clinical test of the set of clinical tests. In particular, said information may be specified by a container-type identifier which is associated to and uniquely identifies said type of container.

The container-type identifiers may be as alphanumeric strings specifying a name or a number or any other notation used for uniquely identifying types of containers. For instance, the alphanumeric string "13x100" refers to a tube having a cross sectional diameter of 13 mm and a longitudinal length of 100 mm. As mentioned above, a type of container may be characterized by one or more shared properties. Examples of shared properties may be shape (e.g. cross-sectional shape, bottom shape - U-shape, V-shape), dimensions (e.g. diameter, length, maximum volume that can be contained), material (e.g. glass, plastic) and type of cap (e.g. colour, shape, closure mechanism). Accordingly, the piece of information specified by a container-type identifier (e.g. the string "13x100") may be construed as a requirement that the container to be selected for holding a collected sample must be of the type associated to the container-type identifier.

Alternatively or additionally, the one or more constraints of the sample container requirement may comprise a type of reagent which is to be present in the container and/or a volume of reagent which is to be present in the container. In particular, said constraints comprise information indicative of a type of reagent which is to be present in the container and/or information indicative of a volume of the reagent which is to be present in the container.

As mentioned above, a sample container may be pre-equipped with one or more reagents that are needed e.g. for preserving the sample or preparing the sample for analysis. In this case, in particular, in order for the result of the test to be reliable, the ratio between the amount of biological sample collected in the container and the given amount of reagent shall lie within a given interval. The information indicative of a type of reagent may be a piece of information comprised in an alphanumeric string specifying a name of the reagent, while the information indicative of the volume of reagent may be a piece of information expressed by a numerical value specifying a quantity of reagent. These pieces of information may be construed as a requirement that the container to be selected for holding a collected sample must contain the prescribed amount of prescribed reagent.

The sample volume requirement may be based on the sample container requirement, e.g. of the type of reagent which is to be present in the container and/or a volume of reagent which is to be present in the container, so that the ratio between the amount of biological sample collected in the container and the given amount of reagent shall lie within a given interval. This way, the reliability of the result of the test(s) of the set of clinical tests is improved.

Summarizing, the step of generating sample-collecting data may comprise determining the type of container that is compatible with, e.g. can be handled, by the instruments that are expected to carry out each clinical test of the set of clinical tests. Exemplarily, the first computing device may check the test data, in particular, the identifier(s) of the set of clinical tests, against the instrument data, in particular the sets of tests that can be performed by each laboratory instrument. Accordingly, the first computing device may identify which instrument(s) can carry out the requested clinical test(s) and look up in the instrument data which tube types are compatible with said instruments and/or which reagent(s) may be required for a given clinical test.

As explained above, sometimes more than one container may be necessary, in which case the sample container requirement comprises not only one or more constraints on a first container but also on at least one additional container. Each container may be configured to receive a sub-volume of the sample. In some examples, the sample container requirement may comprise information indicative of the number of containers, into which the biological sample is to be collected, and information indicative of the type of containers for each container into which the sample container is to be collected. As described above, in this case it is possible to cope with scenarios in which the set of laboratory instruments does not comprise any instrument that can carry out a specific test in the set of clinical tests, so that part of the sample may need to be sent to a different laboratory.

In an example of the first aspect, the step of providing the sample-collecting data to a phlebotomy agent may comprise providing the sample-collecting data to a second computing device that is configured to provide the sample-collecting data to the phlebotomy agent. In particular, the second computing device may be configured to provide by displaying said data on the screen of the second computing device. This is the case if, in particular, the phlebotomy agent is a human being which has access, e.g. can look at, the screen of the first computing device.

For instance, the step of providing the sample-collecting data to the second computing device may be carried out by using at least a computer network, wherein the computer network comprises the first computing device and the second computing device. Exemplarily, the first computing device and the second computing device may be in remote data communication with one another, e.g. via a wireless connection. As discussed above, the first computing device and the second computing device may be part of a LAN or a WAN. The remote data connection may be in particular used for non-sensitive data such as the sample-collecting data. In this case, the generation of the sample-collecting data is streamlined e.g. without jeopardising data protection, as these data do not have to be encoded in an indicium and/or a storage medium that is then materially provided to the phlebotomy agent.

Other data may be sensitive, e.g. subject data that comprise information about at least the identity of the subject. In a particular example, the method may further comprise accessing, by the first computing device, the subject data; wherein the subject data are encoded in an indicium and the indicium is comprised in or arranged on the container. The subject data may be used, e.g. by the second computing device, to prepare result data comprising the results of the test(s) of the set of clinical tests and to provide them to the subject and/or to the physician that has ordered the test(s).

In particular, during the step of accessing the subject data, the container comprises the biological sample. In other words, this step is carried out after the biological sample has been collected in the container and, hence, when the biological sample is moved from the location of the phlebotomy agent to the location of the set of laboratory instruments for testing.

The first computing device may be connected to a reading device configured to access the subject data from the indicium and the step of accessing the subject data may be carried out by using at least the reading device. For example, the indicium may be a barcode and the reading device may be an optical scanner. Alternatively, the subject data may be stored in a RFID tag comprised in or arranged on the container or a rack in which the container is arranged. In this case, the first computing device may comprise or be connected to an RFID reader.

In this way, sensitive data are not transmitted over a network. This may be particularly relevant, for example, if the first computing device is at a location that is remote from the location of the phlebotomy agent and from the location of the physician that ordered the test(s).

Besides the identity of the subject, the subject data may comprise other pieces of information, such as: age, gender, insurance number, vital signs measurements such as blood pressure, medical history including pre-existing conditions.

Exemplarily, the subject data may be encoded in the indicium in an encrypted form and accessing the subject data may comprise decrypting the encrypted subject data. The encryption/decryption may be performed by using an asymmetric encryption algorithm e.g. by means of a pair public key - private key. Alternatively, or in conjunction with the above, encryption/decryption may be performed by using a symmetric encryption algorithm. The encryption increases the security of the transfer of sensitive data.

In some examples of the first aspect, the method may further comprise obtaining, by the first computing device, container data. The container data may comprise at least an identifier of the container, e.g. a universally unique identifier (UUID). The first computing device may obtain the container data from an indicium which is on the container itself or indirectly linked to it, e.g. an indicium and/or a RFID tag in a tube rack configured to accommodate one or more containers. Such tube racks are commonly used when transporting the sample containers from a collecting place to the laboratory. Accordingly, obtaining container data may be particularly relevant, for example, if the first computing device is at the location at which the sample is analysed.

The first computing device may use the obtained container data to identify test data univocally associated to the container data when the clinical tests are about to be performed. In other words, the first computing device may access the test data a first time (e.g. receive them with a request) in an "abstract" way, with the purpose of generating the sample-collecting data, and then access the test data a second time when the test data are linked to an actual sample that has arrived at the laboratory for analysis.

In one example, the first computing device may look up the association between the obtained container data and respective test data in a remote storage at the location at which the phlebotomy agent is located and retrieve the test data therefrom. In another example, the first computing device may have previously received the linked container data/test data e.g. in encrypted form.

Exemplarily, the container data may further comprise information about at least one of: a position of the container on a tube rack, a time at which the container has been inserted in the tube rack, a priority status of the container (i.e. of the sample). These pieces of information may be useful when analysing the sample in the container, e.g. when selecting which container should be gripped by a robotic arm of the ALS for being processed by the laboratory instruments of the ALS and a streamlining the processing of the biological sample is achieved.

In particular, the step of accessing the container data may be carried out by using the aforementioned computer network. Alternatively, or in conjunction with the above, the step of accessing the container data may be carried before the step of accessing the subject data, e.g. when the container is still in the location of the phlebotomy agent. This way, the processing of the biological sample is further streamlined.

In a particular example, the method according to the first aspect may further comprise prompting, by the first computing device, the execution of the at least one clinical test on the biological sample. For instance, the first computing device may be part of the ALS and may control the set of laboratory instruments.

As mentioned, a second aspect of the present invention relates to a computer-implemented method comprising:

- accessing, by a second computing device, sample-collecting data generated based on at least test data and instrument data, wherein:

  o the test data comprise information specifying at least one clinical test to be carried out on a biological sample of a subject,
  o the instrument data comprise information about a state and/or a configuration of a set of laboratory instruments, and at least one laboratory instrument of the set of laboratory instruments is configured to carry out the at least one clinical test;
  o the sample-collecting data comprise at least one of a sample volume requirement and a sample container requirement,
  o the sample volume requirement comprises one or more suitability constraints on a volume of the biological sample, and
  o the sample container requirement comprises one or more constraints on a container in which the biological sample is to be held;

- providing, by the second computing device, the sample-collecting data to a phlebotomy agent.

In a particular example, accessing the sample-collecting data comprises retrieving and/or receiving the sample-collecting data from a first computing device.

Exemplarily, the step of accessing the sample-collecting data may be carried out by using at least a computer network, wherein the computer network comprises the first computing device and the second computing device. Optionally, the first computing device and the second computing device may be in remote data communication with one another.

In a particular example, the method may further comprise encoding, by the second computing device, subject data in an indicium, wherein the subject data comprise information about at least the identity of the subject.

Optionally, encoding the subject data may comprise encrypting the subject data. These operations performed by the second computing device are the counterpart to the operations of the first computing device described above.

Exemplarily, in the context of the present disclosure, three locations may be identified: a first location at which a decision about the clinical tests is made, i.e. at which the clinical tests that shall be carried out on a sample are determined (e.g. a doctor's practice); a second location at which the phlebotomy agent is located, i.e. where a sample is collected, e.g. drawn, from a subject and placed in a sample tube (e.g. a phlebotomist's office); and a third location at which the sample is analysed, i.e. the clinical tests are performed (e.g. a laboratory). Each location may be provided with a computing device.

In some examples, two locations may coincide with each other, e.g. the first location and the second location, for instance if the doctor draws the blood sample themselves, and accordingly the computing device located at the first location is the same as the computing device located at the second location. In other examples, two locations may be physically separated but still within a limited area, such that their computing devices may be distinct but interconnected by a local area network (LAN). This may be the case when, for instance, the phlebotomist office is situated within the doctor's practice or within the laboratory, or if all three locations are within a hospital building. In yet other examples, two locations may be remote with respect to each other and their respective computing devices may be connected by a wide area network (WAN), such as the Internet.

The first computing device may be located at the second location and provide the sample-collecting data directly to the phlebotomy agent via its output unit, e.g. by displaying the sample-collecting data on a screen. Alternatively, if e.g. the first computing device is located at the first location or at the third location and the second computing device is located at the second location, providing the sample-collecting data to a phlebotomy agent may comprise providing the sample-collecting data to a second computing device, wherein the second computing device may in turn provide the sample-collecting data to the phlebotomy agent directly via an output unit. In other examples, the second computing device may be situated at the first location or at the third location.

Exemplarily, the second computing device may be a smartphone or a tablet. Alternatively, e.g. if the phlebotomy agent is a phlebotomy device, the second computing device may be comprised in the phlebotomy device.

If the first computing device is at the first location, the test data may be input by a user, e.g. a doctor, "on the fly", while the instrument data may be retrieved from a data storage located at the (distinct) third location. If the first computing device is at the third location, the test data may be received from a computing device at the first location or second location, while the instrument data may be retrieved from the memory of the first computing device or a local database at the third location.

Another aspect of the present invention relates to a data processing system comprising a processor configured to carry out the methods described above. In particular, the first computing device is a data processing system comprising a processor configured to carry out the method according to the first aspect of the invention. Alternatively, or in conjunction with the above, the second computing may be a data processing system comprising a processor configured to carry out the method according to the second aspect of the invention.

A further aspect of the present invention relates to a laboratory system comprising said data processing system and the set of laboratory instruments.

Yet another aspect of the present invention relates to a computer program product or a computer-readable medium comprising instructions which, when the program is executed by a computer, cause the computer to carry out the methods described above.

According to the present invention, any of the various features of the aspects of the invention may be combined in each of those aspects.

Brief Description of the Drawings

Details of exemplary embodiments are set forth below with reference to the exemplary drawings. Other features will be apparent from the description, the drawings, and from the claims. It should be understood, however, that even though embodiments are separately described, single features of different embodiments may be combined to further embodiments.

Figure 1 shows a schematic representation of an exemplary first computing device and a schematic representation of an exemplary second computing device.

Figure 2 shows a swim lane diagram including exemplary steps performed by the first computing device and the second computing device.

Figure 3 shows an example of instrument data.

Detailed Description

In the following, a detailed description of examples will be given with reference to the drawings. It should be understood that various modifications to the examples may be made. Unless explicitly indicated otherwise, elements of one example may be combined and used in other examples to form new examples.

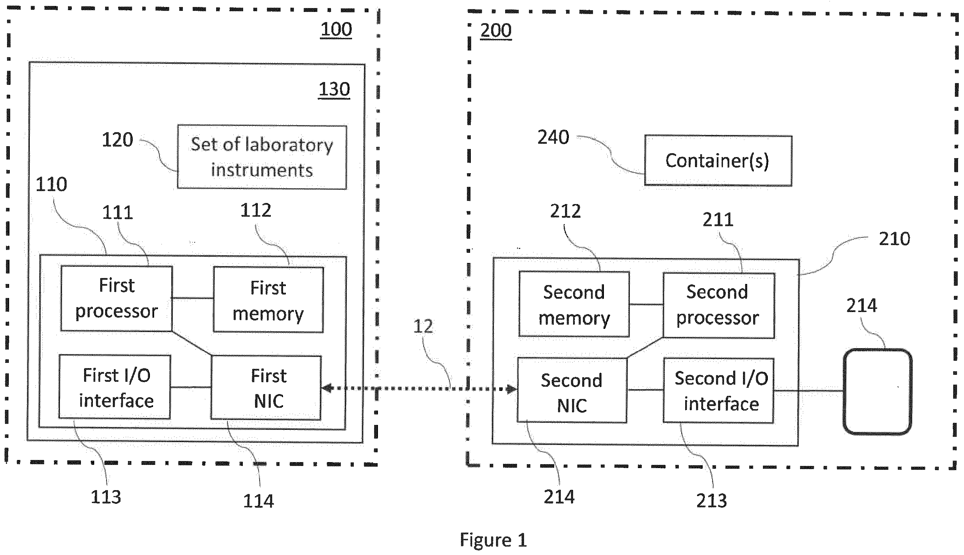

**Figure 1** shows a schematic representation of the first computing device 110 and a schematic representation of the second computing device 210.

The first computing device 110 comprises a first processor 111 (e.g. a CPU, a GPU, or the like), and a first memory 112. The first processor 111 is configured to carry out the method according to the first aspect described above. The first memory 112 may comprise a primary memory and a secondary memory (not shown). In particular, the secondary memory stores a computer program (hereinafter also referred to: "laboratory software") comprising instructions which when executed by the processor 111 cause the first computing device 110 to carry out the method according to the first aspect described above. The first computing device 110 may also comprise a first input output (I/O) interface 113 for communicating with input/output units, such as a screen, a keyboard, a touch screen, a printer, or the like.

The second computing device 210 comprises a second processor 211 (e.g. a CPU, a GPU, or the like), and a second memory 212. The second processor 211 is configured to carry out the method according to the second aspect described above. The second memory 212 may comprise a primary memory and a secondary memory (not shown). In particular, the secondary memory stores a computer program (hereinafter also referred to as: "phlebotomist software") comprising instructions which, when executed by the processor 211, cause the second computing device 210 to carry out the method according to the second aspect described above. The second computing device 210 comprises a second input output (I/O) interface 113 for communicating with a input/output unit, e.g. in the form of a screen 214. In particular, the second computing device 211 may be a laptop or a tablet. If the second computing device 211 is a tablet, the screen 214 is in particular a touch screen.

The first computing device 110 and the second computing device 210 comprise a first Network Interface Controller (NIC) 114 and a second NIC 214, respectively, configured to connect said devices with one or more networks (e.g. an intranet, the internet, a cellular network, or the like). The first computing device 110 and the second computing device 210 may exchange data with one another via the first NIC 114 and the second NIC 214 by using a protocol suite such as TCP or IP, said protocol being schematically represented in Figure 1 by the double-headed dashed line 12.

The first computing device 110 is comprised in an Automated Laboratory System (ALS) 130 that comprises the laboratory instruments (not shown) of the set of laboratory instruments 120. The ALS 130 may comprise transporting means (not shown), e.g. tracks, belts and/or tube containers, or the like, for transporting tubes comprising biological samples to the laboratory instruments for testing. In particular, the first computing device 110 is configured to control the laboratory instruments and the transporting means of the ALS 130. The ALS 130 is located in a laboratory 100.

The second computing device 220 is accessible to and usable by a phlebotomy agent (not shown) which, in this example, is a phlebotomist, e.g. a human being trained to collect, e.g. draw, the biological sample from the subject. The second computing device 210 is located in the phlebotomist's office 200.

Alternatively, the phlebotomy agent may be a phlebotomy device (not shown), e.g. a blood-drawing robot configured to collect, e.g. draw, biological samples from the subject, e.g. by a robotic arm (not shown) configured to draw the biological sample from the subject. In this case, the phlebotomy agent may comprise the second computing device 210, which is configured to control the robotic arm.

Biological samples are placed in a container (or "sample tube") 240, which is subsequently brought to the laboratory 100. The phlebotomy agent may have a plurality of containers 240 at their disposal and, optionally, a plurality of tube racks configured to accommodate one or more containers 240. The tube racks are used for safely and correctly transporting the containers 240. The phlebotomy agent further has the second computing device 220 at their disposal, which is configured to interface with the phlebotomy agent e.g. via the screen 214.

The laboratory 100 receives the biological sample in the container and analyses it e.g. by using the ALS 130. The first computing device 110 and the second computing device 220 are distinct and may be separated by a physical distance that ranges e.g. from the order of metres to the order of kilometres. For example, the phlebotomist's office 200 may be located on site at the laboratory, in which case the first computing device 110 and the second computing device 220 are locally connected, e.g. by means of a LAN. In another example, the phlebotomist's office 200 may be at a physician's office (where the clinical tests are prescribed) that is remote from the laboratory, in which case the two computing devices 110, 220 may communicate remotely over the Internet.

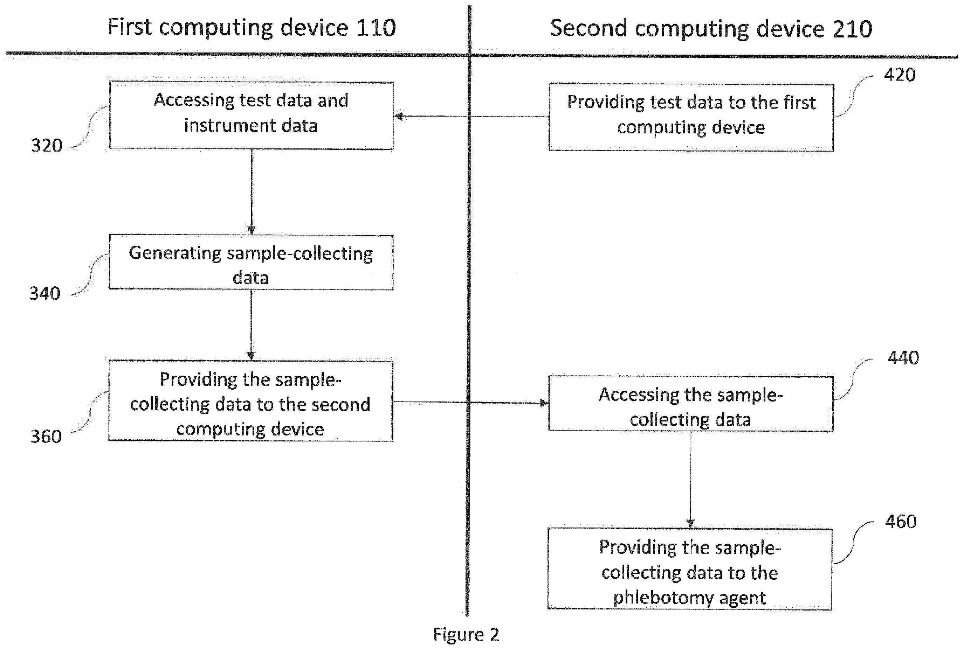

**Figure 2** shows a swim lane diagram including exemplary steps of a method according to the first aspect (steps 320, 340, 360) and exemplary steps of a method according to the second aspect (steps 420, 440, 460). In particular, the first computing device 110 schematically depicted in Figure 1 carries out the steps 320, 340 and 360 and the second computing device 210 carries out the steps 420, 440 and 460.

A physician has their own computing device (a third computing device), which has access to a local database containing the data of the subject (or patient). Further, the third computing device is provided with a piece of software (hereinafter referred to as "physician software") configured to associate one or more clinical tests to a given illness, namely one or more clinical tests needed to validate a given hypothesis about a possible illness.

The physician inputs the given suspected illness (e.g. by giving it in a search field or choosing it from a drop-down list) and the software returns (e.g. after looking it up in a test database) a list of recommended clinical tests. The physician has the option to select one or more among the recommended clinical tests and also add further clinical tests not present in the list. These steps may be repeated for other suspected illnesses.

The final set of clinical tests to be performed for the subject is then imported by the physician from the physician software into the local database and assigned to the subject. In particular, the physician software does not have access to the local database in view of the sensitivity of the patient data.

Finally, the physician prepares the prescription for sample collection from the subject, which is to be carried out either by an in-house phlebotomist or at an external facility (e.g. within a laboratory 100). The prescription contains the test data, in particular the identifiers of the one or more clinical tests to be performed, the subject data and possibly an identifier of the physician. The prescription may be stored in electronic form and sent or made otherwise available to the second computing device 210, or it may be printed and handed out to the subject, who then brings it to the phlebotomist. Exemplarily, the prescription may be encrypted, e.g. using asymmetric cryptography for an electronic prescription or a QR code for a printed prescription.

Thus, the phlebotomist receives the prescription and extracts the test data from it, e.g. by scanning the QR code or opening an encrypted email. The operation of obtaining the test data from the prescription is set off by the phlebotomist software on the second computing device 220. For example, the test data may comprise an alphanumeric string with the identifiers of the clinical tests separated by commas "CA, TSH, T3, T4". "CA" identifies a test that determines the amount of calcium in blood, while "TSH, T3, T4" identify tests that determine a level of the respective thyroid-related hormones.

At step 420, the phlebotomist software instructs the second computing device 210 to provide the test data to the first computing device 110. In particular, step 420 is carried out by sending the test data to the first computing device 110 by using the protocol suite 12.

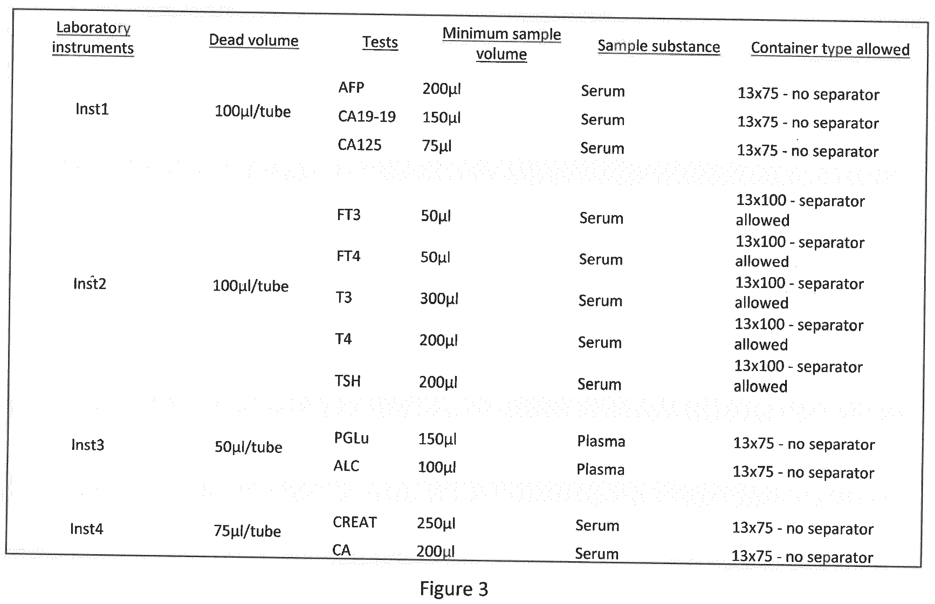

The first computing device 110 accesses at 320 the test data (as received by the second computing device 220) and instrument data. The instrument data are stored in the first memory 112 and/or in a local database of the laboratory 100 and one example thereof is given in **Figure 3.**

**Figure 3** shows a table containing identifiers of laboratory instruments and, for each laboratory instrument, dead volume and a list of clinical tests that can be run by the laboratory instrument, wherein for each test a sample minimum volume, a sample substance and allowed container are indicated.

The first computing device 110 generates at 340 sample-collecting data using at least the test data and the instrument data. The laboratory software checks the clinical tests "CA, TSH, T3, T4" against the set of laboratory instruments and identifies that the laboratory instrument "Inst4" can perform the "CA" test while the laboratory instrument "Inst2" can perform the "TSH", "T3" and "T4" tests. According to the instrument data, for all these tests, serum is required as sample substance.

At step 340, the laboratory software generates the sample volume requirement. In particular, step 340 comprises determining the volume of the biological sample $\overline{V}$, which e.g. is a lower limit for the volume of serum needed, by using equations (1) and (2). In particular, the volume $\overline{V}$ is computed by making the following computation using the values from the instrument data. For the CA test, which will be performed on the Inst4 instrument, the needed amount of serum is given by the minimum sample volume 200 $\mu$l for CA plus the instrument dead volume, (75 $\mu$l), i.e. 275 $\mu$l. For the other three tests, the needed amount of serum is given by the sum of the minimum sample volumes for THS, T4 and T3, namely 200 $\mu$l, 200 $\mu$l and 300 $\mu$l respectively, and the Inst2 dead volume, 100 $\mu$l, which yields 800$\mu$l.

According to the instrument data, the two instruments Inst4 and Inst2 require different container types. Exemplarily, the biological sample may be placed in a single container by the phlebotomist 200 and then aliquoted at the laboratory 100. In this case, the volume of biological sample $\overline{V}$ is computed as the sum of the two subtotals, 1075$\mu$l.

The laboratory software pulls in a value for the serum/blood ratio (e.g. retrieved from the memory of the first computing device 110). A standard serum ratio in blood is 60%, however the value used by the laboratory software may account for individual variations by including a safety leeway of 10% and be set to 50%. Therefore, the volume, $\overline{V}$, of biological sample, i.e. the volume of blood that needs to be drawn from the subject, becomes 1075 $\mu$l/50%= 2150 $\mu$l. Further, an extra buffer of 300 $\mu$l for platelets may be added, bringing the total to 2450 $\mu$l of blood. Accordingly, the first computing device 110 generates, as part of the sample-collecting data, a suitability constraint of "2450 $\mu$l" together with an indication that it represents a lower limit.

Further, since the aliquoting in this case will take place at the laboratory 100, the laboratory software selects either one of the container types allowed for Inst4 and Inst2 as constraint on the container, e.g. "13x100". This alphanumeric string is included in the sample-collecting data, namely as the sample container requirement.

In an alternative example, the incompatibility of the container types allowed by the two instruments may already be addressed at the phlebotomist's side. In this case, the laboratory software generates a sample volume requirement comprising two lower limit constraints, one for the sub-volume meant for the Inst4 and one for the sub-volume meant for the Inst2. Correspondingly, the sample container requirement specifies two container type identifiers, "13x75, 13x100", and the sample-collecting data further indicates which volume is meant for which container. In particular, in this case, the sub-volume that is to be held in the "13x100" container is determined by taking into account the dead volume of Inst2 and the sub-volume that is to be held in the "13x75" container is determined by taking into account the dead volume of Inst4.

At step 360, the sample-collecting data are provided to the second computing device. In particular, this step is carried out by sending the test data to the first computing device 110 by using the protocol suite 12.

At step 440, the second computing device 210 accesses the sample-collecting data. In particular, the phlebotomist software instructs the second computing device 210 to store the sample-collecting data in the second memory 212 and to access said data to provide them to the phlebotomy agent (step 460). In particular, at step 460 the second computing device 210 provides the sample-collecting data to the phlebotomist by displaying said data on the screen 214, e.g. by using a software that generates a GUI (not shown) displaying said data.

The phlebotomist has access to the second computing device 210 and, in particular, can read the requirements comprised in the sample-collecting data by looking at the screen 214, e.g. at the GUI displayed thereon. The information comprised in the sample-collecting data assists the phlebotomist in drawing the biological according to the ALS 130 needs. In this case, in particular, the phlebotomist may place the amount of blood indicated by the sample volume requirement into one or more containers among the available containers 240 according to the sample container requirement.

An identifier, e.g. a numeric string "7213349", is assigned by the second computing device 210 to each sample container, e.g. in the form of a barcode attached to the container. The container identifier is linked to the test data by the phlebotomist software and this information is shared in encrypted form with the first computing device 110, e.g. uploaded in the cloud.

Afterwards, the phlebotomist 200 prepares the sample container(s) holding the sample for transport to the laboratory 100. In particular, the sample container(s) may be arranged in a tube rack comprising an RFID tag and container data about each sample container may be written to the RFID tag by the second computing device 220 (e.g. the second computing device 220 may be connected to an RFID writer). The container data may include the identifier of the sample container, its position in the rack and the priority status of the sample. The RFID tag may also store a tube rack identifier, which may also be shared with the first computing device 110.

Once a sample container arrives at the laboratory, possibly in the tube rack, it is processed by a pre-analytical system or another system connected to the first computing device 110. This system obtains the container data for the first computing device 110, e.g. by means of a scanner, and the first computing device 110 uses the container identifier and/or the tube rack identifier to identify the corresponding test data in the previously received information. The laboratory software uploads a list of all received sample containers to the laboratory information system (LIS), which notes date and time of arrival. The laboratory software also estimates an expected time for the results of the clinical tests and transmits it to the second computing device 220 and/or the third computing device.

The pre-analytical system uses the container data to distribute the tubes according to their priority to the set of laboratory instruments 120. Once the clinical tests are completed and the results have been validated in the LIS, the LIS provides the results to the laboratory software, which transmits them to the physician software and/or the phlebotomist software.

**Claims**

1.  A computer-implemented method comprising:

    - accessing, by a first computing device, test data and instrument data, wherein:

        o the test data comprise information specifying at least one clinical test to be carried out on a biological sample of a subject,
        o the instrument data comprise information specifying a state and/or a configuration of a set of laboratory instruments, and
        ◦ at least one laboratory instrument of the set of laboratory instruments is configured to carry out the at least one clinical test;

    - generating, by the first computing device, sample-collecting data by using at least the test data and the instrument data, wherein:

        ◦ the sample-collecting data comprise at least one of a sample volume requirement and a sample container requirement,
        ◦ the sample volume requirement comprises one or more suitability constraints on a volume of the biological sample, and
        ◦ the sample container requirement comprises one or more constraints on a container in which the biological sample is to be held;

    - providing, by the first computing device, the sample-collecting data to a phlebotomy agent.

2.  The method according to claim 1, wherein providing the sample-collecting data to a phlebotomy agent comprises: providing the sample-collecting data to a second computing device, the second computing device being configured to provide the sample-collecting data to the phlebotomy agent.

3.  The method according to claim 2, wherein the step of providing the sample-collecting data to the second computing device is carried out by using at least a computer network, wherein the computer network comprises the first computing device and the second computing device and, optionally, the first computing device and the second computing device are in remote data communication with one another.

4.  The method according to any one of the preceding claims further comprising:

    accessing, by the first computing device, subject data, wherein the subject data comprise information about at least the identity of the subject;
    wherein the subject data are encoded in an indicium and the indicium is comprised in or arranged on the container.

5.  The method according to claim 4, wherein the subject data is encoded in the indicium in an encrypted form and accessing the subject data comprises decrypting the encrypted subject data.

6.  A computer-implemented method comprising:

    - accessing, by a second computing device, sample-collecting data generated based on at least test data and instrument data, wherein:

        o the test data comprise information specifying at least one clinical test to be carried out on a biological sample of a subject,
        o the instrument data comprise information specifying a state and/or a configuration of a set of laboratory instruments, and at least one laboratory instrument of the set of laboratory instruments is configured to carry out the at least one clinical test;
        ○ the sample-collecting data comprise at least one of a sample volume requirement and a sample container requirement,
        ○ the sample volume requirement comprises one or more suitability constraints on a volume of the biological sample, and
        ○ the sample container requirement comprises one or more constraints on a container in which the biological sample is to be held;

    - providing, by the second computing device, the sample-collecting data to a phlebotomy agent.

7.  The method according to claim 6, wherein accessing the sample-collecting data comprises retrieving and/or receiving the sample-collecting data from a first computing device.

8.  The method according to claim 6 or 7, wherein the step of accessing the sample-collecting data is carried out by using at least a computer network, wherein the computer network comprises the first computing device and the second computing device and, optionally, the first computing device and the second computing device are in remote data communication with one another.

9.  The method according to any one of claims 6 to 8, further comprising:
    encoding, by the second computing device, subject data in an indicium, wherein the subject data comprise information about at least the identity of the subject.

10. The method according to claim 9, wherein encoding the subject data comprises encrypting the subject data.

11. The method according to any one of the preceding claims, wherein:

    the one or more suitability constraints of the sample volume requirement comprise a volume value and/or a volume range;
    and/or, wherein the one or more constraints of the sample container requirement comprise at least one of: a type of container, a type of reagent which is to be present in the container, and
    a volume of reagent which is to be present in the container.

12. A data processing system comprising a processor configured to carry out the method according to any one of the preceding claims.

13. A laboratory system comprising: the data processing system according to claim 12, and the set of laboratory instruments.

14. A computer program product comprising instructions which, when the program is executed by a computer, cause the computer to carry out the method according to any one of claims 1 to 11.

15. A computer-readable medium comprising instructions which, when executed by a computer, cause the computer to carry out the method according to any one of claims 1 to 11.


**Amended claims in accordance with Rule 137(2) EPC.**

1. A computer-implemented method comprising:

   - accessing (320), by a first computing device (110), test data and instrument data, wherein:

     o the test data comprise information specifying at least one clinical test to be carried out on a biological sample of a subject,
     o the instrument data comprise information specifying a state and/or a configuration of a set of laboratory instruments (120), and
     o at least one laboratory instrument of the set of laboratory instruments (120) is configured to carry out the at least one clinical test;

   - generating (340), by the first computing device (110), sample-collecting data by using at least the test data and the instrument data, wherein:

     o the sample-collecting data comprise at least one of a sample volume requirement and a sample container requirement,
     o the sample volume requirement comprises one or more suitability constraints on a volume of the biological sample, and
     o the sample container requirement comprises one or more constraints on a container (240) in which the biological sample is to be held;

   - providing, by the first computing device (110), the sample-collecting data to a phlebotomy agent.

2. The method according to claim 1, wherein providing the sample-collecting data to a phlebotomy agent comprises: providing (360) the sample-collecting data to a second computing device (210), the second computing device (210) being configured to provide the sample-collecting data to the phlebotomy agent.

3. The method according to claim 2, wherein the step of providing (360) the sample-collecting data to the second computing device is carried out by using at least a computer network, wherein the computer network comprises the first computing device (110) and the second computing device (210) and, optionally, the first computing device (110) and the second computing device (210) are in remote data communication with one another.

4. The method according to any one of the preceding claims further comprising:

   accessing, by the first computing device (110), subject data, wherein the subject data comprise information about at least the identity of the subject;
   wherein the subject data are encoded in an indicium and the indicium is comprised in or arranged on the container (240).

5. The method according to claim 4, wherein the subject data is encoded in the indicium in an encrypted form and accessing the subject data comprises decrypting the encrypted subject data.

6. A computer-implemented method comprising:

   - accessing (440), by a second computing device (210), sample-collecting data generated based on at least test data and instrument data, wherein:

     o the test data comprise information specifying at least one clinical test to be carried out on a biological sample of a subject,

o the instrument data comprise information specifying a state and/or a configuration of a set of laboratory instruments (120), and at least one laboratory instrument of the set of laboratory instruments (120) is configured to carry out the at least one clinical test;

o the sample-collecting data comprise at least one of a sample volume requirement and a sample container requirement,

o the sample volume requirement comprises one or more suitability constraints on a volume of the biological sample, and

o the sample container requirement comprises one or more constraints on a container (240) in which the biological sample is to be held;

- providing (460), by the second computing device (210), the sample-collecting data to a phlebotomy agent.

7. The method according to claim 6, wherein accessing (440) the sample-collecting data comprises retrieving and/or receiving the sample-collecting data from a first computing device (110).

8. The method according to claim 7, wherein the step of accessing (440) the sample-collecting data is carried out by using at least a computer network, wherein the computer network comprises the first computing device (110) and the second computing device (210) and, optionally, the first computing device (110) and the second computing device (210) are in remote data communication with one another.

9. The method according to any one of claims 6 to 8, further comprising:
encoding, by the second computing device (210), subject data in an indicium, wherein the subject data comprise information about at least the identity of the subject.

10. The method according to claim 9, wherein encoding the subject data comprises encrypting the subject data.

11. The method according to any one of the preceding claims, wherein:

the one or more suitability constraints of the sample volume requirement comprise a volume value and/or a volume range;

and/or, wherein the one or more constraints of the sample container requirement comprise at least one of: a type of container, a type of reagent which is to be present in the container,

and

a volume of reagent which is to be present in the container.

12. A computing device comprising a processor configured to carry out the method according to any one of the preceding claims.

13. A laboratory system comprising: the computing device according to claim 12, and the set of laboratory instruments, wherein the computing device is operatively connected to the set of laboratory instruments and is configured to control each instrument of the set of laboratory instruments.

14. A computer program product comprising instructions which, when the program is executed by a computer, cause the computer to carry out the method according to any one of claims 1 to 11.

15. A computer-readable medium comprising instructions which, when executed by a computer, cause the computer to carry out the method according to any one of claims 1 to 11.

Figure 1

First computing device 110 | Second computing device 210

Accessing test data and instrument data  ⟵  Providing test data to the first computing device  420

320

Generating sample-collecting data

340

Providing the sample-collecting data to the second computing device  ⟶  Accessing the sample-collecting data  440

360

Providing the sample-collecting data to the phlebotomy agent  460

Figure 2

| Laboratory instruments | Dead volume | Tests | Minimum sample volume | Sample substance | Container type allowed |
|---|---|---|---|---|---|
| Inst1 | 100µl/tube | AFP | 200µl | Serum | 13x75 - no separator |
| | | CA19-19 | 150µl | Serum | 13x75 - no separator |
| | | CA125 | 75µl | Serum | 13x75 - no separator |
| Inst2 | 100µl/tube | FT3 | 50µl | Serum | 13x100 - separator allowed |
| | | FT4 | 50µl | Serum | 13x100 - separator allowed |
| | | T3 | 300µl | Serum | 13x100 - separator allowed |
| | | T4 | 200µl | Serum | 13x100 - separator allowed |
| | | TSH | 200µl | Serum | 13x100 - separator allowed |
| Inst3 | 50µl/tube | PGLu | 150µl | Plasma | 13x75 - no separator |
| | | ALC | 100µl | Plasma | 13x75 - no separator |
| Inst4 | 75µl/tube | CREAT | 250µl | Serum | 13x75 - no separator |
| | | CA | 200µl | Serum | 13x75 - no separator |

Figure 3

## EUROPEAN SEARCH REPORT

**Application Number**

EP 21 19 0794

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/044427 A1 (QUEST DIAGNOSTICS INVEST INC [US]) 24 March 2016 (2016-03-24) * paragraph [0058] * * paragraph [0063] * * paragraph [0031] * * paragraph [0006] – paragraph [0007] * * paragraph [0063] * * paragraph [0063] * * paragraph [0063] * | 1-15 | INV. G16H10/40 |
| A | WO 2020/117780 A1 (BIO RAD LABORATORIES [US]) 11 June 2020 (2020-06-11) * paragraph [0020] * * paragraph [0026] * * paragraph [0042] – paragraph [0044] * | 1-15 | |
| A | WO 2006/053208 A1 (BECTON DICKINSON CO [US]; BACHUR NICHOLAS R JR [US] ET AL.) 18 May 2006 (2006-05-18) * page 3, line 8 – line 27 * * page 14, line 12 – line 24 * | 1-15 | TECHNICAL FIELDS SEARCHED (IPC) G16H |
| A | WO 2011/019576 A1 (SIEMENS HEALTHCARE DIAGNOSTICS [US]; MILLER KERRY L [US]) 17 February 2011 (2011-02-17) * paragraph [0003] * * paragraph [0075] * * paragraph [0078] * | 1-15 | |
| A | WO 2020/035767 A1 (BECKMAN COULTER INC [US]) 20 February 2020 (2020-02-20) * the whole document * | 1-15 | |
| A | EP 3 757 575 A1 (HOFFMANN LA ROCHE [CH]; ROCHE DIAGNOSTICS GMBH [DE]) 30 December 2020 (2020-12-30) * the whole document * | 1-15 | |

The present search report has been drawn up for all claims

| Place of search | Date of completion of the search | Examiner |
|---|---|---|
| Munich | 28 January 2022 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                  

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 0794

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2022

| Patent document cited in search report | | Publication date | Patent family member(s) | | Publication date |
|---|---|---|---|---|---|
| WO 2016044427 | A1 | 24-03-2016 | EP | 3194905 A1 | 26-07-2017 |
| | | | US | 2017292868 A1 | 12-10-2017 |
| | | | WO | 2016044427 A1 | 24-03-2016 |
| WO 2020117780 | A1 | 11-06-2020 | CN | 113167628 A | 23-07-2021 |
| | | | EP | 3870940 A1 | 01-09-2021 |
| | | | KR | 20210088730 A | 14-07-2021 |
| | | | SG | 11202105443P A | 29-06-2021 |
| | | | US | 2021404856 A1 | 30-12-2021 |
| | | | WO | 2020117780 A1 | 11-06-2020 |
| WO 2006053208 | A1 | 18-05-2006 | EP | 1810024 A1 | 25-07-2007 |
| | | | JP | 2008519604 A | 12-06-2008 |
| | | | US | 2006154327 A1 | 13-07-2006 |
| | | | US | 2010003714 A1 | 07-01-2010 |
| | | | US | 2016282330 A1 | 29-09-2016 |
| | | | US | 2019145955 A1 | 16-05-2019 |
| | | | WO | 2006053208 A1 | 18-05-2006 |
| WO 2011019576 | A1 | 17-02-2011 | AU | 2010282784 A1 | 09-02-2012 |
| | | | CA | 2771219 A1 | 17-02-2011 |
| | | | EP | 2464959 A1 | 20-06-2012 |
| | | | JP | 5859439 B2 | 10-02-2016 |
| | | | JP | 6005683 B2 | 12-10-2016 |
| | | | JP | 2013501937 A | 17-01-2013 |
| | | | JP | 2014145774 A | 14-08-2014 |
| | | | US | 2012140230 A1 | 07-06-2012 |
| | | | WO | 2011019576 A1 | 17-02-2011 |
| WO 2020035767 | A1 | 20-02-2020 | NONE | | |
| EP 3757575 | A1 | 30-12-2020 | CN | 112129957 A | 25-12-2020 |
| | | | EP | 3757575 A1 | 30-12-2020 |
| | | | JP | 2021001886 A | 07-01-2021 |
| | | | US | 2020400697 A1 | 24-12-2020 |

EPO FORM P0459

For more details about this annex : see Official Journal of the European Patent Office, No. 12/82